(19) Europäisches Patentamt / European Patent Office / Office européen des brevets

(11) **EP 3 467 533 A1**

(12) **EUROPEAN PATENT APPLICATION**

(43) Date of publication:
**10.04.2019 Bulletin 2019/15**

(51) Int Cl.:
**G01R 33/48** *(2006.01)*     **G01R 33/54** *(2006.01)*

(21) Application number: **17195568.5**

(22) Date of filing: **09.10.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **ETH Zurich**
  **8092 Zürich (CH)**

• **Universität Zürich**
  **8006 Zürich (CH)**

(72) Inventor: **The designation of the inventor has not yet been filed**

(74) Representative: **Schmauder & Partner AG Patent- & Markenanwälte VSP Zwängiweg 7 8038 Zürich (CH)**

(54) **MAGNETIC RESONANCE IMAGING METHOD WITH HYBRID FILLING OF K-SPACE**

(57)     A method for generating an image data set of an image area located in a measurement volume of a magnetic resonance system comprising a gradient system and an RF transmission/reception system, comprises the following method steps:
- reading out k-space corresponding to the imaging area, by:
(a) activating a frequency encoding gradient in a predetermined spatial direction and with a predetermined strength Go by means of said gradient system,
(b) after the activated frequency encoding gradient achieves its strength Go, radiating a non-slice-selective RF excitation pulse by means of said RF transmission/reception system,
(c) after a transmit-receive switch time $\Delta t_1$ following the radiated excitation pulse, acquiring FID signals with said RF transmission/reception system and storing said FID signals as raw data points in k-space along a radial k-space trajectory that is predetermined by the direction and strength Go of the frequency encoding gradient,
(d) repeating (a) through (c) with respectively different frequency encoding gradient directions in each repetition until k-space corresponding to the image area is read out in an outer region of k-space along radial k-space trajectories, said radial k-space trajectories each having a radially innermost limit $k_{gap}$ which depends on said switch time $\Delta t_1$,
(e) reading out a remainder of k-space that corresponds to the imaging area, said remainder being an inner region of k-space not being filled by said first region and including at least a center of k-space, in a read out procedure that is different from (a) through (d), and storing all data points read out in (d) and (e); and

- reconstructing image data from the read out data points in k-space by implementing a reconstruction algorithm.
    In order to constrain image fidelity and optimize scan duration under given circumstances, the inner k-space region is subdivided into a core region and at least one radially adjacent shell region (Si).

Fig. 1

**Description**

<u>Field of the Invention</u>

The present invention generally relates to a method of magnetic resonance (MR) imaging. More specifically, it relates to a zero echo time imaging method useful for MRI of tissues with short coherence lifetimes.

<u>Background of the Invention</u>

Direct imaging of tissues with short transverse relaxation times ($T_2$ or $T_2$*) is interesting both from a scientific and also from a clinical point of view (see [0] and references cited therein). It is known that imaging of samples with relaxation times shorter than a few milliseconds requires specialized imaging methods, which most notably include zero echo time (ZTE) based techniques such as algebraic ZTE, PETRA (Pointwise Encoding Time Reduction with Radial Acquisition) and WASPI (Water And Fat Suppressed Proton Projection MRI) [1-3].

US 8,878,533 B1 is directed to a method for generating an image data set of an image area located in a measurement volume of a magnetic resonance system, the magnetic resonance system comprising a gradient system and an RF transmission/reception system, the method comprising:

- reading out k-space corresponding to the imaging area, by:

    (a) activating at least two phase encoding gradients in respective spatial directions with said gradient system,
    (b) after the activated phase encoding gradients achieve a full strength, radiating a non-slice-selective RF excitation pulse with said RF transmission/reception system,
    (c) after a time t1 following the radiated excitation pulse, acquiring echo signals with said RF transmission/reception system and storing said echo signals as raw data points in k-space along a radial k-space trajectory that is predetermined by the strength of the phase encoding gradients,
    (d) repeating (a) through (c) with respectively different phase encoding gradients in each repetition until k-space corresponding to the image area is read out in a first region of k-space along radial k-space trajectories, depending on said time t1, and
    (e) reading out a remainder of k-space that corresponds to the imaging area, said remainder not being filled by said first region and including at least a center of k-space, in a read out procedure that is different from (a) through (d), and storing all data points read out in (d) and (e); and

- reconstructing image data from the read out data points in k-space by implementing a reconstruction algorithm in a computerized processor, the reconstruction algorithm comprising a Fourier transformation of the data points.

According to the above, the k-space region corresponding to the imaging area is subdivided into an inner region and an outer region, wherein the outer region surrounds the inner region, the inner region containing the center of k-space. Different read out procedures are used in the inner and outer regions. In the outer region, the read out is conveniently done along radial k-space trajectories using a gradient strength Go, whereas in the inner region some different read out procedure is adopted.

According to an advantageous embodiment defined in US 8,878,533 B1, the raw data points in the inner region are acquired as Cartesian raw data. This implementation is generally known as the PETRA technique.

A somewhat different approach is used in the case of WASPI, where the raw data in the inner region are also acquired along radial k-space trajectories, but with a lower gradient strength $G_w < G_0$. The two datasets, i.e. the inner dataset and the outer dataset are combined with optional linear merging in an overlap region [0].

While the use of algebraic ZTE is favorable at small dead-time gaps, direct measurement of missing data is required when more than 3 Nyquist dwells are missed. In this case, PETRA is usually preferred over WASPI due to its robustness against short-T2 related artifacts [4]. However, its scan efficiency decreases quickly with gap size.

Accordingly, it would be desirable to provide an improved MR imaging method. It is thus an object of the present invention to provide a zero echo time imaging method useful for MRI of tissues with short coherence lifetimes which can overcome some of the limitations of the presently used methods such as algebraic ZTE, WASPI and PETRA.

<u>Summary of the Invention</u>

According to the present invention, a method for generating an image data set of an image area located in a measurement volume of a magnetic resonance system comprising a gradient system and an RF transmission/reception system, comprises the following method steps:

- reading out k-space corresponding to the imaging area, by:

(a) activating a frequency encoding gradient in a predetermined spatial direction and with a predetermined strength Go by means of said gradient system,
(b) after the activated frequency encoding gradient achieves its strength Go, radiating a non-slice-selective RF excitation pulse by means of said RF transmission/reception system,
(c) after a transmit-receive switch time $\Delta t_1$ following the radiated excitation pulse, acquiring FID signal with said RF transmission/reception system and storing said FID signal as raw data points in k-space along a radial k-space trajectory that is predetermined by the direction and strength Go of the frequency encoding gradient,
(d) repeating (a) through (c) with respectively different frequency encoding gradient directions in each repetition until k-space corresponding to the image area is read out in an outer region of k-space along radial k-space trajectories, said radial k-space trajectories each having a radially innermost limit $k_{gap}$ which depends on said switch time $\Delta t_1$,
(e) reading out a remainder of k-space that corresponds to the imaging area, said remainder being an inner region of k-space not being filled by said first region and including at least a center of k-space, in a read out procedure that is different from (a) through (d), and storing all data points read out in (d) and (e); and

- reconstructing image data from the read out data points in k-space by implementing a reconstruction algorithm.

By virtue of the fact that the inner k-space region is subdivided into a core region and at least one radially adjacent shell region Si, with raw data points in the core region being acquired as Cartesian raw data, and raw data points in the shell region $S_i$ being acquired along radial k-space trajectories using a gradient strength $G_i$ that is smaller than the gradient strength Go, it is possible to optimize image fidelity and scan duration under given circumstances.

The method of the present invention will henceforth be addressed as hybrid filling and abbreviated as "HYFI". The HYFI approach improves scan efficiency at large gaps with minimum loss of image quality.

The method of the present invention can be implemented in a conventional MR imaging apparatus.

Further details and definitions are given in the section "Theory" further below.

Advantageous embodiments are defined in the dependent claims.

According to one embodiment (claim 2), wherein the boundary $k_{gap}$ subdividing the inner and outer k-space regions is given by the product of bandwidth BW and dead time $\Delta t_0$, wherein the dead time $\Delta t_0$ is given by $\Delta t_{RF}$, which is a part of the RF pulse, particularly half of the RF pulse for symmetric RF pulses, plus the transmit-receive switch time $\Delta t_1$.

According to a further embodiment (claim 3), the core region has an outer limit $k_{core}$ given by

$$k_{core} = \frac{\Delta t_0}{\Delta t} \cdot s_{Min}$$

wherein

- the dead time $\Delta t_0$ is given by $\Delta t_{RF}$, which is a part of the RF pulse, particularly half of the RF pulse for symmetric RF pulses, plus the transmit-receive switch time $\Delta t_1$,
- the allowed acquisition duration $\Delta t$ is given by $-T_2 \ln(1-A)$ wherein A is an amplitude parameter selected between 0 and 1.

With the above definition, one can limit Cartesian acquisition to a small core region and thus allow using the more efficient radial acquisition wherever possible.

According to another embodiment (claim 4), the shell region comprises at least two shell regions ($S_1$, $S_2$, ...), each shell region Si having a shell thickness $s_i$ given by

$$s_i = \frac{\Delta t}{\Delta t_0} \cdot k_{in}$$

each shell region having an inner radius defined by the thickness of the next radially inward core or shell region. Starting from the innermost region, i.e. the core region, concentric shell regions are added in an onion-like manner until reaching the boundary between inner k-space region and outer k-space region. In an alternative embodiment (claim 5), the various shell regions are radially overlapping, in which case the signal in the overlap region is subjected to a linear interpolation step.

According to yet another embodiment (claim 6), the reconstruction algorithm comprises a Fourier transformation of the data points.

Brief description of the drawings

The above mentioned and other features and objects of this invention and the manner of achieving them will become more apparent and this invention itself will be better understood by reference to the following description of embodiments of this invention taken in conjunction with the accompanying drawings, wherein:

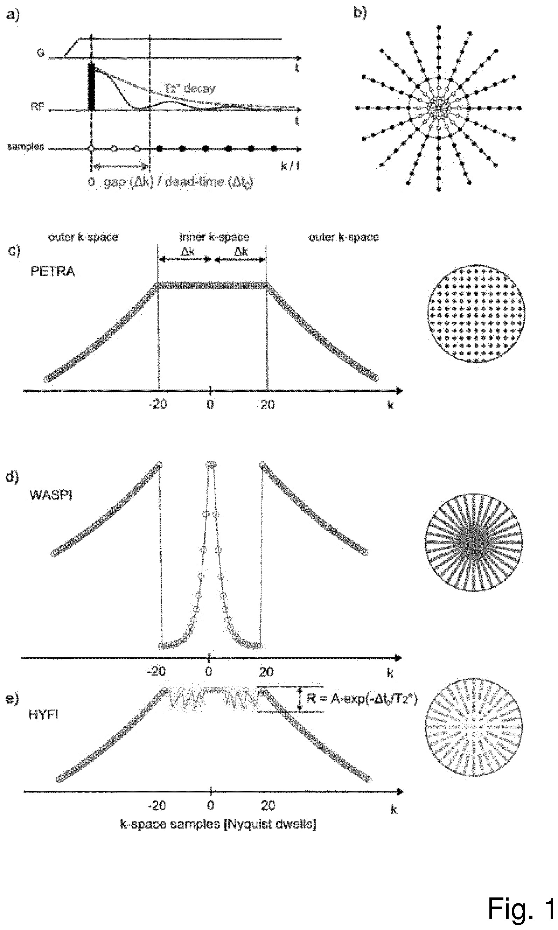

Fig. 1    shows data acquisition in zero echo time-based techniques:

a) the gradient is ramped up before spin excitation; data located around the k-space center (white dots) cannot be acquired because of the dead-time gap ($\Delta t_0$);
b) between excitations, the gradient direction is changed slowly to acquire different projections in order to fill the 3D k-space volume of interest of which a central 2D plane is shown;
c-e) PETRA, WASPI, and HYFI differ in the way they recover the missing data; Left: 1 D depiction of k-space T2* weighting; Right: 2D depiction of inner k-space acquisition geometry;
c) in PETRA, the inner k-space is acquired single-pointwise in a Cartesian fashion leading to constant T2* weighting;
d) in WASPI, a second set of radial acquisitions is performed at lower gradient strength, giving rise to strong T2* weighting;
e) HYFI, the method of this invention, is a hybrid between PETRA and WASPI: a combination of Cartesian SPI and multiple sets of radial acquisitions is used to keep the T2* decay in a given range R, wherein R

is chosen such as to allow higher scan efficiency with minimum loss of image quality;

Fig. 2    shows relevant timing intervals in relation to the applied RF pulse; $\Delta t_0$ includes a part of the pulse, half of it for symmetric pulses and the transmit-receive switch time $\Delta t_1$; and $\Delta t$ is the time window corresponding to the acquisition of each shell;

Fig. 3    shows some fundamental relations holding for a given temporal decay of transversal magnetization $M_{xy}(t)$;

Fig. 4    shows an example of HYFI acquisition wherein the inner region of-k space is subdivided into a core region surrounded by two radially adjacent shell regions s1 and s2; the outer region of k-space is not shown in this figure, but it generally surrounds the second shell region s2;

Fig. 5    shows the number of excitations required by each technique to fill the inner k-space, assuming a T2* decay of 64 Nyquist dwells; circles at the top illustrate the acquisition geometries; in 3D, the number of excitations evolves with gap^3 for PETRA and gap^2 for WASPI; in the proposed method, inner k-space is filled by a combination of SPI and radial acquisitions, and thus the green area enclosed by the curves for PETRA and WASPI becomes accessible; green lines represent selected HYFI acquisitions with amplitude coefficients A = 0.1 and 0.3; and

Fig. 6    shows a simulation of point spread functions: 1 D HYFI acquisitions were simulated with the following fixed parameters: image matrix size = 128, T2* = 64 Nyquist dwells. PSFs are displayed for different combinations of gap sizes (a-c) and amplitude coefficients A (0 = PETRA, 1 = WASPI).

Detailed description of the invention

*Theory*

The HYFI principle is described schematically in Fig.1 in comparison with the known methods of PETRA and WASPI. Fig. 1 a shows the time course of an applied gradient G (upper trace), the irradiated RF pulse and the resultant FID signal (middle trace), and positions of acquired samples in time t and k-space, respectively (lower trace). Also shown in Fig. 1 a is the so-called "gap" $\Delta k$ (in k-space) or "dead time" $\Delta t_0$ (in time) during which data acquisition is blinded due to hardware artifacts.

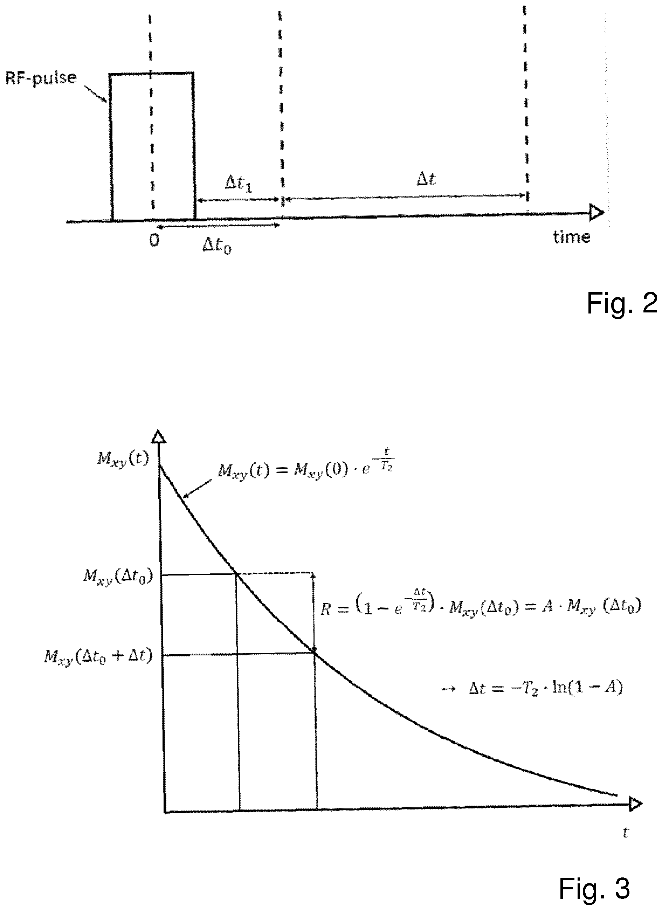

As shown in Fig. 2, the dead time $\Delta t_0$ (counted from the center of the RF pulse) is given by a part of the pulse, half of it for symmetric pulses, plus the transmit-receive switch time $\Delta t_1$ of the MRI apparatus.

In order to avoid short-T2 related artifacts, T2* decay in the inner k-space is limited to a range given by

$$R = A \cdot \exp(-\frac{\Delta t_0}{T_2^*})$$

with the amplitude coefficient $A \in [0,1]$ and the dead-time, $\Delta t_0$. The choice of the amplitude coefficient A will depend on certain external requirements and may need to be optimized in preliminary data acquisitions. Typically, A may be selected in the range of 0.1 to 0.3.

The inner k-space region is filled by a combination of Cartesian and radial acquisitions performed at lower gradient strengths.

Acquiring data at the inner k-space region requires that

$$k(\Delta t_0, G) < k(\Delta t_0, G_0) = \Delta k$$

with $k = \gamma G t$, $\gamma$ the gyromagnetic ratio [Hz/T], G the gradient used for the acquisition of the k-space center [T/m], $\Delta t_0$ the dead time [s], $G_0$ the gradient used for acquisition of the outer k-space [T/m].

Hence we must fulfill the following relation

$$G < G_0$$

It should be noted that:

- a smaller gradient means smaller k-space velocity $v_k$ (in units of $m^{-1} \cdot s^{-1}$)

$$v_k = \frac{dk}{dt} = \gamma G$$

- moving more slowly in k-space increases T2-weighting of the data (i.e. the amplitude decays faster for a given k-space range).

It should also be noted that T2-weightings diverge from pure exponential decay, and point spread functions diverge from Lorentzians.

The point spread function (PSF) of PETRA is preferred to the PSF of WASPI in view of better image fidelity due to smaller side lobes, but PETRA acquisitions are significantly longer at large gaps. The number of shots required for PETRA (Cartesian acquisition) and WASPI (radial acquisition) to fill the gap is:

$$n_{PETRA} \approx \frac{4}{3} \cdot \pi \cdot gap^3$$

whereas

$$n_{WASPI} \approx 4 \cdot \pi \cdot gap^2$$

Based on the above, the principle of the HYFI method may be summarized as follows:

- Make radial acquisitions whenever possible to optimize scan efficiency.
- Limit the allowed T2-decay to a given range, R, by limiting acquisition to a certain duration ($\Delta t$), to avoid the appearance of strong side lobes in the PSF.
- In this manner HYFI allows the optimization of scan duration while constraining depiction fidelity.

Accordingly, k-space is acquired in a shell-like manner. The core or ($0^{th}$ shell) is acquired single pointwise on a Cartesian grid and is surrounded by shells that are acquired radially.

- The shell thickness, s, is given by the allowed acquisition duration $\Delta t$ and the k-space acquisition velocity $v_k$.

$$s = \Delta t \cdot v_k = \Delta t \cdot \gamma \cdot G \; [m^{-1}]$$

- In units of Nyquist dwells [dw], the shell surface is calculated as follow

$$s = \Delta t \cdot v_k \cdot FOV = \Delta t \cdot \gamma \cdot G \cdot FOV = \Delta t \cdot BW \; [\text{ dw }]$$

With BW the imaging bandwidth.
- The k-space acquisition velocity, $v_k$, is proportional to the gradient G (see above) which is determined by inner radius of the shell, $k_{in}$.

$$\gamma \cdot G \cdot \Delta t_0 = k_{in} \; [m^{-1}] \qquad or \qquad \gamma \cdot G \cdot \Delta t_0 \cdot FOV = k_{in} \; [dw]$$

$$\rightarrow G = \frac{k_{in}}{\gamma \cdot \Delta t_0 \cdot FOV}$$

with $k_{in}$ in Nyquist dwells.
- With all this, we can rewrite the shell thickness, s, as follow

$$s = \frac{\Delta t}{\Delta t_0} \cdot \text{k}_{in}$$

Note: shell thickness increases linearly with inner radius.
- The radius of the core, $k_{core}$ is calculated from the minimum thickness required to do radial acquisitions, $s_{Min}$ (which is typically taken as 1 Nyquist dwell (lower limit to acquire 2 points)).

$$k_{core} = \frac{\Delta t_0}{\Delta t} \cdot s_{Min}$$

$$Acquisition\ geometry = \begin{cases} Cartesian\ SPI, & k \leq k_{core} \\ radial, & k > k_{core} \end{cases}$$

- The number of radial spokes to be acquired for the jth shell is:

$$4 \cdot \pi \cdot k^2_{out_j}$$

With $k_{out_j}$ the outer shell radius. $k_{out_j} = k_{in_j} + s_j$. With $k_{in_j}$ the inner radius of the jth shell.
- Shells boundary condition:

$$k_{in_1} = k_{core}$$

$$k_{out_j} = k_{in_j} + s_j$$

$$k_{in_{j+1}} = k_{out_j} + \delta k \qquad \text{with } 0 \leq \delta k \leq 1$$

Note:

∘ if δ*k* = 0, Nyquist criterion is fulfilled everywhere.
∘ If δ*k* = 1, Nyquist criterion is not fulfilled at shell boundaries.

As an option, it would also be possible to linearly merge shells over an overlap region in order to decrease irregularities in the k-space weighting.
HYFI step by step:

1) Define targeted transverse relaxation time T2
2) Define maximum allowed decay range R within the shell (choose amplitude parameter A between 0 and 1).
3) Calculate maximum acquisition duration

$$\Delta t = -T_2 \cdot \ln(1 - A)$$

4) Define $s_{Min}$, typically chosen to be 1 dw.
5) Calculate core radius, $k_{core}$, to be filled with SPI.

$$k_{core} = \frac{\Delta t_0}{\Delta t} \cdot s_{Min}$$

6) Calculate inner and outer radius for each shell for each $k_{in_j} < gap$

$$k_{in_1} = k_{core}$$

$$k_{out_j} = k_{in_j} + s_j$$

$$k_{in_{j+1}} = k_{out_j} + \delta k \qquad \text{with } 0 \leq \delta k \leq 1$$

7) Calculate k-space directions

   a. SPI

      i. All k-space point on Cartesian grid fulfilling

$$|k| \leq k_{core}$$

   b. Radial

      i. Calculate number of shots (or radial spokes) to fulfill Nyquist criterion on the outer surface of each shell j

$$n = 4 \cdot \pi \cdot k_{out_j}^2$$

      ii. Calculate the directions of each shot by spreading these points on the surface of each shell following a suitable trajectory with approximately equal density.

8) Measure data on calculated trajectory
9) Reconstruct image

*Concluding remarks*

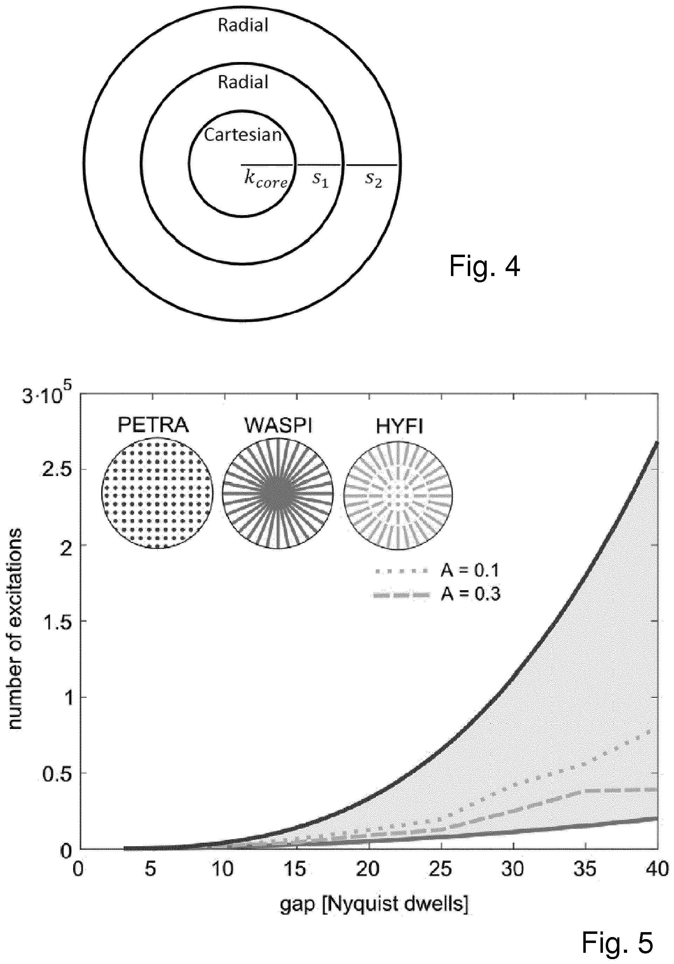

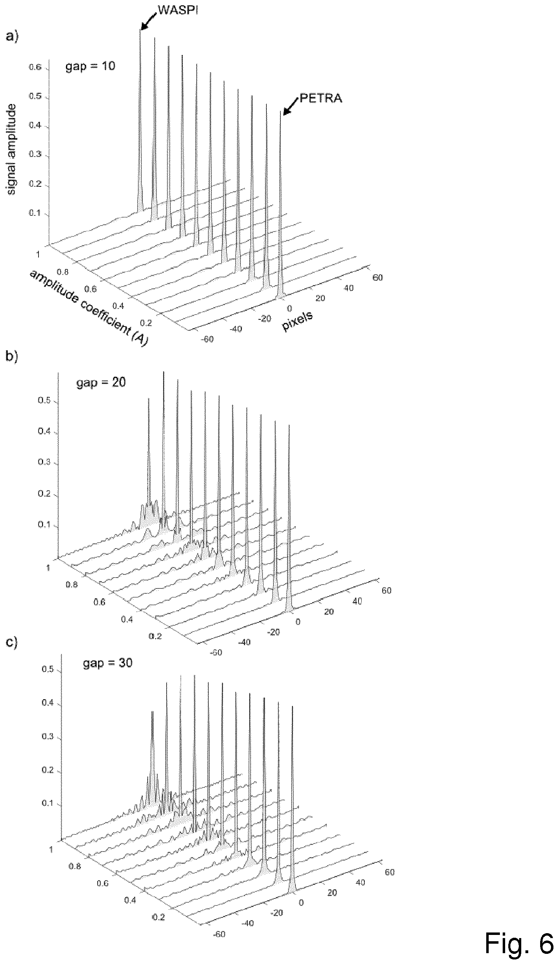

Fig. 5 illustrates the scan efficiency of the different methods. At large gaps, the number of excitations required to fill the inner k-space is significantly larger in PETRA (4/3*π*gap^3) than in WASPI (4*pi*gap^2). In HYFI, relatively low amplitude coefficients (0.1 to 0.3) allow significant reduction of the number of excitations compared to PETRA while preserving satisfactory PSF lineshapes (Fig. 6). For example, at a gap of 30 Nyquist dwells and a T2* of 64 Nyquist dwells, the use of HYFI with A = 0.3 reduces the number of shots required to fill the inner k-space by almost 80% (from $1.15 \cdot 10^5$ to $2.45 \cdot 10^4$). Assuming repetition times between 1 and 10 ms, this leads to a net gain of 1.5 to 15 minutes per scan.

In the following, the HYFI method is evaluated with 1 D simulations of point spread functions (PSF), see Fig. 6.

One-dimensional point spread functions (PSF) were simulated by application of the following formula:

$$P = F \cdot T \cdot E \cdot \delta_0$$

wherein F is the pseudo inverse of the encoding matrix E, T is the T2* weighting matrix (T2* = 64 Nyquist dwells) and $\delta_0$ is the Kronecker delta function located in the center of the field of view.

*References*

[0] Froidevaux, R. et al. (2017), Filling the dead-time gap in zero echo time MRI: Principles compared. Magn Reson Med. 2017 Aug 30. doi: 10.1002/mrm.26875.

[1] Wu Y, Dai G, Ackerman JL, Hrovat MI, Glimcher MJ, Snyder BD, Nazarian A, Chesler D a. Water- and fat-suppressed proton projection MRI (WASPI) of rat femur bone. Magn Reson Med 2007;57:554-67.

[2] Grodzki DM, Jakob PM, Heismann B. Ultrashort echo time imaging using pointwise encoding time reduction with radial acquisition (PETRA). Magn Reson Med 2012;67:510-8.

[3] Weiger M, Pruessmann KP. MRI with Zero Echo Time. eMagRes 2012;1:311-22.

[4] Froidevaux R, Weiger M, Brunner DO, Wilm BJ, Dietrich BE, Pruessmann KP. Filling the dead time gap in zero echo time MRI : principles compared. Proc 25th Annu Meet ISMRM, Singapore 2016: 3298.

[5] Froidevaux R, Weiger M, Rösler MB, Wilm B, Hennel F, Luechinger R, Dietrich BE, Reber J, Pruessmann KP. Ultra-high-bandwidth , high-resolution MRI of fast relaxing spins Shot-T2 MRI : requirements High resolution. Proc 26th Annu Meet ISMRM, Honolulu 2017:4037.

## Claims

1. A method for generating an image data set of an image area located in a measurement volume of a magnetic resonance system, the magnetic resonance system comprising a gradient system and an RF transmission/reception system, the method comprising:

   - reading out k-space corresponding to the imaging area, by:

      (a) activating a frequency encoding gradient in a predetermined spatial direction and with a predetermined strength Go by means of said gradient system,
      (b) after the activated frequency encoding gradient achieves its strength Go, radiating a non-slice-selective RF excitation pulse by means of said RF transmission/reception system,
      (c) after a transmit-receive switch time $\Delta t_1$ following the radiated excitation pulse, acquiring FID signals with said RF transmission/reception system and storing said FID signals as raw data points in k-space along a radial k-space trajectory that is predetermined by the direction and strength Go of the frequency encoding gradient,
      (d) repeating (a) through (c) with respectively different frequency encoding gradient directions in each repetition until k-space corresponding to the image area is read out in an outer region of k-space along radial k-space trajectories, said radial k-space trajectories each having a radially innermost limit $k_{gap}$ which depends on said switch time $\Delta t_1$,
      (e) reading out a remainder of k-space that corresponds to the imaging area, said remainder being an inner region of k-space not being filled by said first region and including at least a center of k-space, in a read out procedure that is different from (a) through (d), and storing all data points read out in (d) and (e); and

   - reconstructing image data from the read out data points in k-space by implementing a reconstruction algorithm;

   **characterized in that**

the inner k-space region is subdivided into a core region and at least one radially adjacent shell region ($S_i$), with raw data points in the core region being acquired as Cartesian raw data, and raw data points in the shell region (Si) being acquired along radial k-space trajectories using a gradient strength $G_i$ that is smaller than the gradient strength Go.

2. The method according to claim 1, wherein the boundary $k_{gap}$ subdividing the inner and outer k-space regions is given by the product of bandwidth BW and dead time $\Delta t_0$, wherein the dead time $\Delta t_0$ is given by $\Delta t_{RF}$, which is a part of the RF pulse, particularly half of the RF pulse for symmetric RF pulses, plus the transmit-receive switch time $\Delta t_1$.

3. The method according to claim 1 or 2, wherein the core region has an outer limit kcore given by

$$k_{core} = \frac{\Delta t_0}{\Delta t} \cdot s_{Min}$$

wherein

- the dead time $\Delta t_0$ is given by $\Delta t_{RF}$, which is a part of the RF pulse, particularly half of the RF pulse for symmetric RF pulses, plus the transmit-receive switch time $\Delta t_1$,
- the allowed acquisition duration $\Delta t$ is given by $-T_2 \ln(1-A)$ wherein A is an amplitude parameter selected between 0 and 1.

4. The method according to one of claims 1 to 3, wherein the shell region comprises at least two shell regions ($S_1$, $S_2$, ...), each shell region $s_i$ having a shell thickness $s_i$ given by

$$s_i = \frac{\Delta t}{\Delta t_0} \cdot k_{in}$$

each shell region having an inner radius defined by the thickness of the next radially inward core or shell region.

5. The method according to one of claims 1 to 3, wherein the shell region comprises at least two shell regions ($S_1$, $S_2$, ...), each shell region Si having a shell thickness $s_i$ given by

$$s_i = \frac{\Delta t}{\Delta t_0} \cdot k_{in}$$

each pair of adjacent shell regions having a radial overlap region, wherein signal in each overlap region is obtained by linear signal merging.

6. The method according to one of claims 1 to 5, wherein the reconstruction algorithm comprises a Fourier transformation of the data points.

Fig. 1

Fig. 2

$$M_{xy}(t) = M_{xy}(0) \cdot e^{-\frac{t}{T_2}}$$

$$R = \left(1 - e^{-\frac{\Delta t}{T_2}}\right) \cdot M_{xy}(\Delta t_0) = A \cdot M_{xy}(\Delta t_0)$$

$$\rightarrow \quad \Delta t = -T_2 \cdot \ln(1 - A)$$

Fig. 3

Fig. 4

Fig. 5

Fig. 6

Europäisches
Patentamt

European
Patent Office

Office européen
des brevets

# EUROPEAN SEARCH REPORT

Application Number

EP 17 19 5568

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2008/265885 A1 (DANNELS WAYNE R [US]) 30 October 2008 (2008-10-30) * the whole document * | 1-6 | INV. G01R33/48 G01R33/54 |
| A | US 2017/261577 A1 (SMINK JOUKE [NL] ET AL) 14 September 2017 (2017-09-14) * the whole document * | 1-6 | |
| A | HYUNGSEOK JANG ET AL: "Ramped hybrid encoding for improved ultrashort echo time imaging", MAGNETIC RESONANCE IN MEDICINE., vol. 76, no. 3, 18 September 2015 (2015-09-18), pages 814-825, XP055445765, US ISSN: 0740-3194, DOI: 10.1002/mrm.25977 * the whole document * | 1-6 | |
| A,D | ROMAIN FROIDEVAUX ET AL: "Filling the dead-time gap in zero echo time MRI: Principles compared", MAGNETIC RESONANCE IN MEDICINE., 30 August 2017 (2017-08-30), XP055445737, US ISSN: 0740-3194, DOI: 10.1002/mrm.26875 * the whole document * | 1-6 | TECHNICAL FIELDS SEARCHED (IPC) G01R |
| A,D | US 8 878 533 B2 (GRODZKI DAVID [DE]; HEISMANN BJOERN [DE]; SIEMENS AG [DE]) 4 November 2014 (2014-11-04) * the whole document * | 1-6 | |

-/--

The present search report has been drawn up for all claims

| Place of search | Date of completion of the search | Examiner |
|---|---|---|
| Munich | 29 March 2018 | Raguin, Guy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 17 19 5568

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | DAVID M. GRODZKI ET AL: "Ultrashort echo time imaging using pointwise encoding time reduction with radial acquisition (PETRA)", MAGNETIC RESONANCE IN MEDICINE, vol. 67, no. 2, 30 June 2011 (2011-06-30), pages 510-518, XP055027471, ISSN: 0740-3194, DOI: 10.1002/mrm.23017 * the whole document * | 1-6 | |
| A | M. Weiger ET AL: "MRI with Zero Echo Time" In: "Encyclopedia of Magnetic Resonance", 15 June 2012 (2012-06-15), John Wiley & Sons, Ltd, Chichester, UK, XP055168958, ISBN: 978-0-47-003459-0 DOI: 10.1002/9780470034590.emrstm1292, * the whole document * | 1-6 | |
| A,D | YAOTANG WU ET AL: "Water- and fat-suppressed proton projection MRI (WASPI) of rat femur bone", MAGNETIC RESONANCE IN MEDICINE, vol. 57, no. 3, 1 January 2007 (2007-01-01), pages 554-567, XP055039080, ISSN: 0740-3194, DOI: 10.1002/mrm.21174 * the whole document * | 1-6 | TECHNICAL FIELDS SEARCHED (IPC) |

The present search report has been drawn up for all claims

| Place of search | Date of completion of the search | Examiner |
|---|---|---|
| Munich | 29 March 2018 | Raguin, Guy |

EPO FORM 1503 03.82 (P04C01)

## ANNEX TO THE EUROPEAN SEARCH REPORT
## ON EUROPEAN PATENT APPLICATION NO.

EP 17 19 5568

This annex lists the patent family members relating to the patent documents cited in the above-mentioned European search report.
The members are as contained in the European Patent Office EDP file on
The European Patent Office is in no way liable for these particulars which are merely given for the purpose of information.

29-03-2018

| Patent document cited in search report | | Publication date | Patent family member(s) | | Publication date |
|---|---|---|---|---|---|
| US 2008265885 | A1 | 30-10-2008 | EP | 1851563 A2 | 07-11-2007 |
| | | | JP | 5042862 B2 | 03-10-2012 |
| | | | JP | 2008529643 A | 07-08-2008 |
| | | | US | 2008265885 A1 | 30-10-2008 |
| | | | WO | 2006120584 A2 | 16-11-2006 |
| US 2017261577 | A1 | 14-09-2017 | CN | 107076818 A | 18-08-2017 |
| | | | EP | 3191862 A1 | 19-07-2017 |
| | | | JP | 2017530761 A | 19-10-2017 |
| | | | US | 2017261577 A1 | 14-09-2017 |
| | | | WO | 2016038048 A1 | 17-03-2016 |
| US 8878533 | B2 | 04-11-2014 | DE | 102010041446 A1 | 29-03-2012 |
| | | | US | 2012074938 A1 | 29-03-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8878533 B1

**Non-patent literature cited in the description**

- **FROIDEVAUX, R. et al.** Filling the dead-time gap in zero echo time MRI: Principles compared. *Magn Reson Med.,* 30 August 2017
- **WU Y ; DAI G ; ACKERMAN JL ; HROVAT MI ; GLIMCHER MJ ; SNYDER BD ; NAZARIAN A ; CHESLER D A.** Water- and fat-suppressed proton projection MRI (WASPI) of rat femur bone. *Magn Reson Med,* 2007, vol. 57, 554-67
- **GRODZKI DM ; JAKOB PM ; HEISMANN B.** Ultrashort echo time imaging using pointwise encoding time reduction with radial acquisition (PETRA). *Magn Reson Med,* 2012, vol. 67, 510-8
- **WEIGER M ; PRUESSMANN KP.** MRI with Zero Echo Time. *eMagRes,* 2012, vol. 1, 311-22

- **FROIDEVAUX R ; WEIGER M ; BRUNNER DO ; WILM BJ ; DIETRICH BE ; PRUESSMANN KP.** Filling the dead time gap in zero echo time MRI : principles compared. *Proc 25th Annu Meet ISMRM,* 2016, 3298
- **FROIDEVAUX R ; WEIGER M ; RÖSLER MB ; WILM B ; HENNEL F ; LUECHINGER R ; DIETRICH BE ; REBER J ; PRUESSMANN KP.** Ultra-high-bandwidth , high-resolution MRI of fast relaxing spins Shot-T2 MRI : requirements High resolution. *Proc 26th Annu Meet ISMRM,* 2017, 4037